# EUROPEAN PATENT SPECIFICATION

(11) **EP 1 973 483 B1**
(45) Date of publication and mention of the grant of the patent: **03.04.2019**
(21) Application number: 06840231.2
(22) Date of filing: 13.12.2006
(51) Int. Cl.: A61B 17/70

(54) **VERTEBRAL ROD ASSEMBLIES**
WIRBELSTANGENANORDNUNGEN
ENSEMBLES TIGES VERTEBRALES

(30) Priority: 20.12.2005 US 313000
(43) Date of publication of application: 01.10.2008
(73) Proprietor: Warsaw Orthopedic, Inc., Warsaw, IN 46581 (US)
(72) Inventor: NULL, William B., Olive Branch, Mississippi 38654 (US); DREWY, Troy D., Memphis, Tennessee 38117 (US)
(74) Representative: HGF Limited
(86) International application number: PCT/US2006/061998
(87) International publication number: WO 2007/076265

(56) References cited:
- EP-A- 0 953 316
- FR-A1- 2 781 359
- US-A1- 2002 138 077
- US-A1- 2004 015 166
- US-A1- 2005 228 376

## Description

### BACKGROUND

The present application relates generally to spinal implant assemblies, and more particularly to a vertebral rod system having first and second connectable and adjustable members.

The spine is divided into four regions comprising the cervical, thoracic, lumbar, and sacrococcygeal regions. The cervical region includes the top seven vertebrae identified as C1-C7. The thoracic region includes the next twelve vertebral identified as T1-T12. The lumbar region includes five vertebrae L I-L5. The sacrococcygeal region includes nine fused vertebrae that form the sacrum and the coccyx. The vertebrae of the spine are aligned in a curved configuration that includes a cervical curve, thoracic curve, and lumbosacral curve.

Vertebral rods may be implanted to support and position the vertebrae in. one or more of these regions. The rods extend along a section of the spine and are connected to the vertebrae with one or more fasteners. The rods may have a curved configuration to conform to the curvature of the spine. Often times two or more rods are connected together and work in combination to support and position the vertebrae. The rods may have the same or different shapes and sizes depending upon their position along the spine.

EP0953316 describes a bone fixation device with transverse linking bridge in which coupling hooks clip on to bars 2, 3 on which bone fixation elements are located.

US2005/228376 A1 describes a spinal fixation device having first and second elongate members, which members are angularly adjustable relative to one another. A locking mechanism comprising a male connecting part, a female connecting part and a fastener lock the elongate members in a fixed position relative to one another

### Summary

The present application relates to a vertebral rod assembly as defined in the claims. In one embodiment, the vertebral rod assembly comprises first and second members that may rotate with respect to each other about a first axis. Each of the first and second members includes a base and a vertebral support rod extending from the base. The base on the first member includes an extension member connected thereto, which extension member extends into an opening formed in the base of the second member. A fastener extends through a sidewall of the base of the second member and into the opening to lock the first and second members, and to prevent the rotation of the first member and second member.

### Detailed Description

The present application is directed to vertebral rod assemblies, which, according to the invention, has first and second members. First and second members are selectively positionable at a variety of angles to conform to the curvature of the patient's spine. Each member includes a rod and a base. A locking device locks the position of the first and second members at the desired orientation to prevent further movement.

The first member has a rod and a base. The rod is attached to the base and extends outward therefrom. According to the invention, the rod and base are integrally formed together. The base includes a first surface having a plurality of splines spaced thereabout forming a series of alternating ridges and valleys. The splines cover at least a portion of the first surface. In one embodiment, the splines are spaced about the periphery, and extend radially inward from an outer edge of the first surface towards a central point. In another embodiment, the splines extend outwards from about the central point of the first surface towards the periphery.

An extension extends outward from the first surface of the base of the first member and includes a shaft having an angled surface that flares outward to form an enlarged head. The angled surface of the shaft of the extension slopes outward away from the first surface of the base of the first member and terminates at a flat outer surface of the head of the extension. A rim may be positioned at the peripheral edges of the angled surface and the head.

According to the invention the second member has a rod and a base. As with the first member, the rod of the second member is integrally formed with the base of the second member and extends outward therefrom. The base of the second member includes a first surface having a plurality of splines formed as alternating ridges and valleys. The splines on the second member are formed to correspond to the size and spacing of the splines on the first member. A receiving section extends from the first surface of the base of the second member into the interior of the base of the second member and is sized to receive the extension. One or more openings are formed in a sidewall of the base of the second member and extend into the interior of the base. In one embodiment, a pair of sidewall openings are positioned in the base of the second member and spaced apart by about 180° (i.e., the sidewall openings are on opposite sides of the base of the second member). Multiple openings allow for easier access during the locking and unlocking procedures as will be discussed below. In one embodiment, the one or more openings in the sidewall of the base of the second member may be threaded.

A fastener extends through the sidewall opening formed in the base of the second member. In one embodiment, the fastener is a screw having threads that engage the threads formed on the sidewall opening. The fastener inserts into the sidewall opening, and has a contact surface that contacts against the surface of the extension of the first member. The surface of the extension and the contact surface of the fastener have corresponding shapes to increase the area of contact between the fastener and extension when the first and second members are coupled in a locked configuration. The contact surface at the end of the fastener is angled and corresponds to the angle of surface of the extension. In other embodiments, however, the surface of the extension and the contact surface of the fastener may not have corresponding shapes. In still other embodiments, the fastener contact surface and/or the inserted end of the fastener are shaped to engage the shaft of the extension.

Placing the first and second members in a locked configuration prevents the first and second members from rotating relative to each other about an axis of rotation (hereinafter referred to as "the first axis"). To rotate the first and second members about the first axis, the assembly may be placed in an unlocked configuration as described below in more detail.

The extension of the base of the first member inserts into the receiving section formed in the base of the second member. When the extension is fully inserted, the splines of the first and second members engage and interlock with each other. Once engaged, the interlocking splines prevent the relative rotation of the first and second members. The fastener inserts into the sidewall opening and is rotated about an axis R₂ (hereinafter referred to as "the second axis") until the fastener engages the second member. In a particular embodiment, the fastener inserts into the sidewall opening and is rotated about the second axis until the contact surface of the fastener engages the angled surface of the extension. Continuing to rotate the fastener about the second axis causes the contact surface of the fastener to apply a force to the angled surface of the extension, which force locks the second base to the first base. This prevents the separation and rotation of the first and second members. This force also maintains the splines in the interlocking relationship. In this embodiment, the second axis is substantially perpendicular to the first axis. In other embodiments, the second axis is substantially non-parallel to the first axis.

To place the assembly in the unlocked configuration, in one embodiment, the fastener is rotated about the second axis such that contact surface of the fastener moves away from surface of the extension. This movement releases the locking force that the contact surface of the fastener applies to the angled surface of the extension. This movement also allows for disengaging the interlocked splines. Once the splines are disengaged, the first and/or second members may be rotated relative to each other by rotating first and/or second bases about the first axis. It is not necessary to separate the contact surface of the fastener from the angled surface of the extension completely, nor is it necessary to remove the fastener from the base of the second member altogether. Rather, it is enough to rotate the fastener a sufficient amount to reduce the force applied by the contact surface of the fastener to the angled surface of the extension and to disengage the splines such that the first and second members may rotate relative to one another.

Multiple openings within the sidewall of the base of the second member provide additional options for locking the device. Positioning the members along the patient's spine at the proper angle may result in one of the sidewall openings being blocked. In this event, the fastener may be inserted into a subsequent sidewall opening that is accessible. Additionally, more than one fastener may be used for engagement with a different fastener inserted within each of the sidewall openings

In an embodiment, the assembly comprises an end cap coupled to the assembly using a mechanical, fastener. As in the previous embodiment, the first and second members are selectively positionable at a variety of angles to conform the first and second rods of the first and second members generally to the curvature of the patient's spine.

In an embodiment, the surface of the base of the first member comprises splines formed as a plurality of alternating ridges and valleys that extend radially inward from a sidewall of the first base. The splines are spaced inward from the sidewall of the base of the first member, forming a generally flat edge surface that extends around the periphery of the base of the first member. The shaft of the extension comprises a substantially cylindrical member that connects to and extends outward from the surface of the base of the first member. A threaded opening at one end of the shaft of the extension extends into the shaft. The threaded opening is formed to receive the mechanical fastener that secures the end cap to the assembly.

The shaft of the extension also includes a tab extending from its exterior sidewall. The tab includes a ramped surface that terminates at an additional surface perpendicular to the shaft of the extension. A stop extends outward beyond the perpendicular surface and has one or more opposing contact surfaces. As described below in more detail, the tab comprises part of the locking device that locks the position of the first and second members at the desired orientation and may limit the angle through which the first and second members may rotate.

In one example, the second member includes a surface having splines comprising a plurality of ridges and valleys that extend radially inward towards the center of the base of the second member. The size and spacing of the splines corresponds generally to the splines of the first member. A generally flat edge surface extends between the splines of the second member and the peripheral edge of the base of the second member. When the first member is mated to the second member, the splines of the second member extend into the base of the first member to engage with the splines of the first member. Additionally, the flat edge surface between the splines and peripheral edge of the base of the second member contacts the corresponding flat edge surface on the base of the first member.

The second member further comprises an opening with a notch formed in the surface of the second member. The opening and the notch open into a receiving section of the second member. The opening and the notch are sized to receive the shaft and the tab of the extension, respectively, when the first member mates with the second member. One or more openings are formed in a sidewall of the base of the second member and extend into the receiving section of the second member. The sidewall openings may be disposed at any desired position on the sidewall of the base of the second member. As described in more detail below, the fastener extends through the sidewall opening to lock the first and second members.

According to the invention the assembly further comprises a ring that fits within the receiving section of the second member and extends around the shaft of the extension. The ring is a substantially cylindrical member that includes an outer sidewall and an inner sidewall that form a cavity. A notch is formed on the inner sidewall of the ring to receive the tab extending from the shaft of the extension. An angled surface slopes upward from a peripheral edge of the inner sidewall of the ring and is angled to correspond to the angle of the ramped surface of the tab extending from the shaft of the extension. The angled surface of the ring also extends around the inner circumference of the ring from the notch of the ring to a stop surface. A threaded opening extends through the ring into the cavity of the ring and is sized to receive the fastener.

In one embodiment, the stop surface is formed at one end of the angled surface of the ring. The stop surface contacts the stop on the tab to control the extent of relative rotation of the first and second members. During rotation, the ramped surface of the tab may slide along angled surface of the ring until the contact surface of the stop on the tab engages the stop surface. In one embodiment, the first and second members rotate through an angle of approximately 180°. However, those skilled in the art will readily appreciate that the stop surface may be formed on the ring at any position such that the first and second members may only rotate a predetermined distance in one direction to limit the rotation.

Placing the first and second members in the locked configuration prevents the first and second members from rotating relative to each other about the first axis. During assembly, the ring is positioned within the receiving section of the second member, with the threaded opening in the ring aligning with the sidewall opening in the base of the second member. An outer diameter of the ring is smaller than an inner diameter of the receiving section of the second member, such that the ring can move along the first axis within the receiving section of the second member. Further, the shaft of the extension inserts into the opening of the second member with the tab aligning with the notch in the opening and the notch in the ring. The fastener is initially inserted into the sidewall openings of the ring and of the base of the second member.

Prior to tightening the fastener, the first and second members are rotated to position the rods of the first and second members. Once oriented, the fastener is tightened causing the ring to move along the first axis within the receiving section of the second member towards the fastener. This movement, in turn, causes the angled surface on the ring to contact and apply a locking force to the ramped surface on the tab. Adjusting the fastener within the sidewall openings of the ring and of the base of the second member adjusts the degree of the locking force and thus prevents further rotation of the first and second members.

When the first and second members are positioned together, the splines formed on base of the second member engage and interlock with the splines formed on the base of the first member. This engagement further prevents rotation of the first and second members. In addition, peripheral fiat edge surfaces of the first and second members contact each other to form a substantially smooth seam extending around the circumference of the first and second bases.

The end cap may be connected to assembly using a screw. The end cap is sized to extend across the receiving section of the base of the second member. The end cap may further include a gasket to sealingly engage the sidewall of the base of the second member and seal the receiving section of the base of the second member.

In yet another embodiment, the shaft of the extension terminates at a head. Both the shaft and the head have a substantially constant width that form a "T".
The fastener includes a contact surface that engages the shaft to lock the device. The contact surface substantially matches the shaft to increase the contact area.

The fastener may further be positioned for an edge to contact the head of the extension to further lock the device. In one embodiment, the locking force is provided between the contact surface of the fastener and the shaft. In another embodiment, the locking force is provided between the edge of the fastener and the head of the extension.

I It should be noted that the base of the first member and/or the base of the second member may have other shapes and sizes, .Further, the shapes and sizes of the bases may be the same or may be different Likewise, the various openings may have different shapes and sizes to receive one or more various sizes and/or types of fasteners.

Each of the rods of the first and second members may have variety of lengths and diameters. The lengths and/or diameters of one or both of the rods may be the same or different. Moreover, one or both of the rods may be integrally formed with the sidewalls of their respective bases, or formed separately and attached to the sidewalls of their respective bases. Further, the splines on the first and second members may extend continuously around their respective bases or, alternatively, may be formed in sections that are spaced apart along the surfaces of the first and second bases.

In one embodiment, the first and second axes of rotation (R₁ and R₂) are substantially perpendicular, as has been described hereinbefore. In other embodiments, however, the first and second axes are aligned at a variety of angular orientations. Further, the second axis may be aligned to be substantially parallel with one or both of the rods of the first and second members. In other embodiments, the second axis may be positioned in a non-parallel manner.
The present invention may be carried out in other ways than those specifically set forth herein without departing from essential characteristics of the invention. A seal may be placed on one or both bases of the first and second members, to sealingly engage the bases together in the locked configuration. The present embodiments are to be considered in all respects as described and not restrictive, and all changes coming within the scope of the appended claims are intended to be embraced therein.

## Claims

1. A vertebral rod assembly comprising:
a first member having a first base and a first vertebral rod extending outward therefrom;
a second member having a second base and a second vertebral rod extending outward therefrom; and
a fastener to lock the second base to the first base to prevent rotation of the first member relative to the second member, the fastener being spaced away from the first and second vertebral rods,
wherein the first vertebral rod is integrally formed with the first base, and the second vertebral rod is formed integrally with the second base,
**characterised in that**
the first member further comprises an extension extending outward from the first base and into the second base, the extension having a first end connected to the first base and a second end spaced away from the first base,
wherein the fastener extends from the second base and into contact with the extension
a receiving section formed within the second base that is sized to receive the extension extending from the first base;
a ring disposed within the receiving section and sized to extend around the extension; and
said fastener extending through the second base and the ring to lock the second base to the first base to prevent rotation of the first and second members
wherein the extension comprises a tab having a first contact surface that extends outward from a side of the extension, and wherein the ring comprises a second contact surface that engages the first contact surface to lock the first and second bases.

2. The assembly of claim 1 further comprising a plurality of interlocking features formed on each of the first and second bases that engage together when the fastener locks the second base to the first base.

3. The assembly of claim 1, wherein the fastener extends through the second base and into the receiving section, the fastener having a fastener contact surface that contacts the extension at a point spaced away from the first end to lock the second base to the first base and prevent the first member from rotating relative to the second member.

4. The assembly of claim 3, wherein the extension comprises a flared section having a width that is larger than the first end, the fastener contact surface contacting the flared section.

5. The assembly of claim 4, wherein the fastener contact surface is angled to substantially match the flared section of the extension to increase a contact area between the fastener and the extension.

6. The assembly of claim 3 further comprising first and second openings formed in the second base each sized to receive the fastener, the first and second openings being spaced apart,

7. The assembly of claim 6, wherein the fastener is positioned within the first opening and a second fastener is positioned within the second opening, each of the first and second fasteners sized to contact the extension.

8. The assembly of claim 3 wherein the first base and the second base sealingly engage to seal the extension within the receiving section, and optionally wherein the assembly further comprises splines on the first and second bases that engage together when the first and second bases are connected together.

9. The assembly of claim 1 further comprising an end cap mounted to the extension and sized to extend over the receiving section.

10. The assembly of claim 1 or 9, wherein the first and second contact surfaces have substantially the same angle to slidingly engage each other when the first member rotates relative to the second member.

11. The assembly of claim 1 or 9, wherein the fastener engages a threaded opening in the ring to cause the second contact surface to apply a force to the first contact surface to lock the second base to the first base.

12. The assembly of claim 1 or 9 further comprising a stop surface positioned along an inner surface of the ring that contacts the tab to control an extent of rotation between the first and second members.

13. The assembly of claim 1 further comprising a first opening formed in the second base and a second opening formed in the ring, wherein the first and second openings align to receive the fastener.

14. The assembly of claim 1, wherein said fastener extends into the second base and the ring at a point spaced from the first and second vertebral rods, the fastener applying a force to the ring to lock the second base to the first base.

15. The assembly of claim 14, wherein the ring comprises a contact surface along an inner peripheral edge that contacts the extensions to lock the ring to the first member.

16. The device of claim 15, wherein the contact surface and the extension each have complementary angled surfaces that contact together to increase a contact area.

## Patentansprüche

1. Wirbelstangenanordnung, umfassend:
ein erstes Element mit einer ersten Basis und einer sich davon nach außen erstreckenden ersten Wirbelstange;
ein zweites Element mit einer zweiten Basis und einer sich davon nach außen erstreckenden zweiten Wirbelstange; und
einen Befestiger zum Verriegeln der zweiten Basis mit der ersten Basis, um eine Drehung des ersten Elements relativ zum zweiten Element zu verhindern, wobei der Befestiger von der ersten und der zweiten Wirbelstange beabstandet ist,
wobei die erste Wirbelstange einstückig mit der ersten Basis geformt ist und die zweite Wirbelstange einstückig mit der zweiten Basis geformt ist,
**dadurch gekennzeichnet, dass**
das erste Element ferner eine sich von der ersten Basis nach außen und in die zweite Basis erstreckende Verlängerung, wobei die Verlängerung ein mit der ersten Basis verbundenes erstes Ende und ein von der ersten Basis beabstandetes zweites Ende aufweist,
wobei sich der Befestiger von der zweiten Basis und in Kontakt mit der Verlängerung erstreckt,
wobei in der zweiten Basis ein Aufhahmeabschnitt geformt ist, der so größenbemessen ist, dass er die sich von der ersten Basis erstreckende Verlängerung aufnimmt;
einen im Aufhahmeabschnitt angeordneten und so größenbemessenen Ring, dass er sich um die Verlängerung erstreckt; und
den sich durch die zweite Basis und den Ring erstreckenden Befestiger, um die zweite Basis mit der ersten Basis zu verriegeln, um eine Drehung des ersten und zweiten Elements zu verhindern, umfasst,
wobei die Verlängerung eine Lasche mit einer ersten Kontaktfläche aufweist, die sich von einer Seite der Verlängerung nach außen erstreckt, und wobei der Ring eine zweite Kontaktfläche aufweist, die die erste Kontaktfläche in Eingriff nimmt, um die erste und zweite Basis zu verriegeln.

2. Anordnung nach Anspruch 1, ferner umfassend eine Vielzahl von Verzahnungsmerkmalen, die auf jeder der ersten und zweiten Basis geformt sind, die einander in Eingriff nehmen, wenn der Befestiger die zweite Basis mit der ersten Basis verriegelt.

3. Anordnung nach Anspruch 1, wobei sich der Befestiger durch die zweite Basis und in den Aufnahmeabschnitt erstreckt, wobei der Befestiger eine Befestigerkontaktfläche aufweist, die die Verlängerung an einer vom ersten Ende beabstandeten Stelle kontaktiert, um die zweite Basis mit der ersten Basis zu verriegeln und das erste Element am Drehen relativ zum zweiten Element zu hindern.

4. Anordnung nach Anspruch 3, wobei die Verlängerung einen ausgestellten Abschnitt mit einer Breite, die größer als das erste Ende ist, aufweist, wobei die Befestigerkontaktfläche den ausgestellten Abschnitt kontaktiert.

5. Anordnung nach Anspruch 4, wobei die Befestigerkontaktfläche so abgewinkelt ist, dass sie dem ausgestellten Abschnitt der Verlängerung im Wesentlichen entspricht, um eine Kontaktfläche zwischen dem Befestiger und der Verlängerung zu vergrößern.

6. Anordnung nach Anspruch 3, ferner umfassend erste und zweite Öffnungen, die in der zweiten Basis geformt und jeweils so größenbemessen sind, um den Befestiger aufzunehmen, wobei die ersten und zweiten Öffnungen beabstandet sind.

7. Anordnung nach Anspruch 6, wobei der Befestiger in der ersten Öffnung positioniert ist und ein zweiter Befestiger in der zweiten Öffnung positioniert ist, wobei jeder des ersten und des zweiten Befestigers so größenbemessen ist, dass er die Verlängerung kontaktiert.

8. Anordnung nach Anspruch 3, wobei sich die erste Basis und die zweite Basis dichtend in Eingriff nehmen, um die Verlängerung im Aufhahmeabschnitt abzudichten, und optional, wobei die Anordnung ferner Kerben an der ersten und der zweiten Basis umfasst, die einander in Eingriff nehmen, wenn die erste und zweite Basis miteinander verbunden werden.

9. Anordnung nach Anspruch 1, ferner umfassend eine Endkappe, die an der Verlängerung montiert und so größenbemessen ist, dass sie sich über den Aufnahmeabschnitt erstreckt.

10. Anordnung nach Anspruch 1 oder 9, wobei die erste und die zweite Kontaktfläche im Wesentlichen denselben Winkel aufweisen, um einander gleitend in Eingriff zu nehmen, wenn sich das erste Element relativ zum zweiten Element dreht.

11. Anordnung nach Anspruch 1 oder 9, wobei der Befestiger eine Gewindeöffnung im Ring in Eingriff nimmt, um die zweite Kontaktfläche zu veranlassen, eine Kraft auf die erste Kontaktfläche anzulegen, um die zweite Basis mit der ersten Basis zu verriegeln.

12. Anordnung nach Anspruch 1 oder 9, ferner umfassend eine entlang einer Innenfläche des Rings positionierte Anschlagfläche, die die Lasche kontaktiert, um ein Ausmaß an Drehung zwischen dem ersten und dem zweiten Element zu steuern.

13. Anordnung nach Anspruch 1, ferner umfassend eine erste in der zweiten Basis geformte Öffnung und eine zweite im Ring geformte Öffnung, wobei die erste und die zweite Öffnung aufeinander ausgerichtet sind, um den Befestiger aufzunehmen.

14. Anordnung nach Anspruch 1, wobei sich der Befestiger in die zweite Basis erstreckt und der Ring an einer Stelle von der ersten und zweiten Wirbelstange beabstandet ist, wobei der Befestiger eine Kraft an den Ring anlegt, um die zweite Basis mit der ersten Basis zu verriegeln.

15. Anordnung nach Anspruch 14, wobei der Ring eine Kontaktfläche entlang einer Innenperipheriekante umfasst, die die Verlängerung kontaktiert, um den Ring mit dem ersten Element zu verriegeln.

16. Vorrichtung nach Anspruch 15, wobei die Kontaktfläche und die Verlängerung jeweils komplementäre abgewinkelte Flächen aufweisen, die einander kontaktieren, um eine Kontaktfläche zu vergrößern.

## Revendications

1. Ensemble tige vertébrale comprenant :
un premier organe comportant une première base et une première tige vertébrale s'étendant vers l'extérieur de celle-ci ;
un second organe comportant une seconde base et une seconde tige vertébrale s'étendant vers l'extérieur de celle-ci ; et
une pièce de fixation pour verrouiller la seconde base à la première base pour empêcher la rotation du premier organe par rapport au second organe, la pièce de fixation étant espacée des première et seconde tiges vertébrales,
ladite première tige vertébrale étant formée d'une seule pièce avec la première base et ladite seconde tige vertébrale étant formée d'une seule pièce avec la seconde base,
**caractérisé en ce que**
le premier organe comprend en outre une extension s'étendant vers l'extérieur de la première base et dans la seconde base, l'extension possédant une première extrémité reliée à la première base et une seconde extrémité espacée de la première base,
ladite pièce de fixation s'étendant depuis la seconde base et en contact avec l'extension
une section de réception formée à l'intérieur de la seconde base qui est dimensionnée pour recevoir l'extension s'étendant depuis la première base ;
une bague disposée à l'intérieur de la section de réception et dimensionnée pour s'étendre autour de l'extension ; et
ladite pièce de fixation s'étendant à travers la seconde base et la bague pour verrouiller la seconde base à la première base pour empêcher la rotation des premier et second organes
ladite extension comprenant une patte possédant une première surface de contact qui s'étend vers l'extérieur d'un côté de l'extension, et ladite bague comprenant une seconde surface de contact qui vient au contact de la première surface de contact pour verrouiller les première et seconde bases.

2. Ensemble selon la revendication 1 comprenant en outre une pluralité d'éléments d'interverrouillage formés sur chacune des première et seconde base qui viennent en contact les uns avec les autres lorsque la pièce de fixation verrouille la seconde base à la première base.

3. Ensemble selon la revendication 1, ladite pièce de fixation s'étendant à travers la seconde base et dans la section de réception, ladite pièce de verrouillage possédant une première surface de contact de pièce de verrouillage qui est en contact avec l'extension au niveau d'un point espacé de la première extrémité pour verrouiller la seconde base à la première base et empêcher le premier organe de tourner par rapport au second organe.

4. Ensemble selon la revendication 3, ladite extension comprenant une section évasée présentant une largeur qui est plus grande que la première extrémité, la surface de contact de pièce de fixation étant en contact avec la section évasée.

5. Ensemble selon la revendication 4, ladite surface de contact de pièce de fixation étant inclinée pour correspondre sensiblement à la section évasée de l'extension pour augmenter la zone de contact entre la pièce de fixation et l'extension.

6. Ensemble selon la revendication 3 comprenant en outre des première et seconde ouvertures formées dans la seconde base dimensionnées chacune pour recevoir la pièce de fixation, les première et seconde ouvertures étant espacées l'une de l'autre.

7. Ensemble selon la revendication 6, ladite pièce de fixation étant positionnée à l'intérieur de la première ouverture et une seconde pièce de fixation étant positionnée à l'intérieur de la seconde ouverture, chacun des première et seconde pièces de fixation étant dimensionnées pour être en contact avec l'extension.

8. Ensemble selon la revendication 3, ladite première base et ladite seconde base venant en contact étanche pour sceller l'extension dans la section de réception, et éventuellement ledit ensemble comprenant en outre des cannelures sur les première et seconde bases qui viennent en contact les unes avec les autres lorsque les première et seconde bases sont reliées l'une à l'autre.

9. Ensemble selon la revendication 1 comprenant en outre un embout monté sur l'extension et dimensionné pour s'étendre sur la section de réception.

10. Ensemble selon la revendication 1 ou 9, lesdites première et seconde surfaces de contact possédant sensiblement le même angle pour s'engager de manière coulissante l'une avec l'autre lorsque le premier organe tourne par rapport au second organe.

11. Ensemble selon la revendication 1 ou 9, ladite pièce de fixation s'engageant dans une ouverture filetée dans la bague pour inciter la seconde surface de contact à appliquer une force sur la première surface de contact pour verrouiller la seconde base à la première base.

12. Ensemble selon la revendication 1 ou 9, comprenant en outre une surface supérieure positionnée le long d'une surface interne de la bague qui est en contact avec la patte pour contrôler l'ampleur de la rotation entre les premier et second organes.

13. Ensemble selon la revendication 1 comprenant en outre une première ouverture formée dans la seconde base et une seconde ouverture formée dans la bague, lesdites première et seconde ouvertures s'alignant pour recevoir la pièce de fixation.

14. Ensemble selon la revendication 1, ladite pièce de fixation s'étendant dans la seconde base et la bague au niveau d'un point espacé des première et seconde tiges vertébrales, la pièce de fixation appliquant une force sur la bague pour verrouiller la seconde base à la première base.

15. Ensemble selon la revendication 14, ladite bague comprenant une surface de contact le long d'un bord périphérique intérieur qui est en contact avec l'extension pour verrouiller la bague au premier organe.

16. Dispositif selon la revendication 15, ladite surface de contact et ladite extension comportant chacune des surfaces inclinées complémentaires qui sont en contact les unes avec les autres pour augmenter la zone de contact.
